# EUROPEAN PATENT SPECIFICATION

(11) **EP 1 485 346 B1**
(45) Date of publication and mention of the grant of the patent: **08.10.2008**
(21) Application number: 02758897.9
(22) Date of filing: 22.07.2002
(51) Int. Cl.: C07C 237/12, A61K 9/127, C12N 15/88

(54) **NOVEL POSITIVELY CHARGED LIPIDS AND LIPOSOMES COMPRISING THE POSITIVELY CHARGED LIPIDS**
NEUE POSITIV GELADENE LIPIDE UND LIPOSOMEN, DIE DIE POSITIV GELADENEN LIPIDE ENTHALTEN
NOUVEAUX LIPIDES POSITIVEMENT CHARGES ET LIPOSOMES COMPRENANT CES LIPIDES POSITIVEMENT CHARGES

(30) Priority: 20.03.2002 KR 2002014923
(43) Date of publication of application: 15.12.2004
(73) Proprietor: Yonsei University, Seoul 120-749 (KR)
(72) Inventor: PARK, Yong Serk, 220-042 Wonju, Kangwon-do (KR); KIM, Hong Sung, 760-090 Andong, Gyeongsangbuk-do (KR); MOON, Jae Ho, Kangnam-gu, 135-110 Seoul (KR); JANG, Doo Ok, 220-042 Wonju, Kangwon-do (KR); HEO, Yeon, 133-080 Seoul (KR)
(74) Representative: Duckworth, Timothy John
(86) International application number: PCT/KR2002/001377
(87) International publication number: WO 2003/078383

(56) References cited:
- WO-A-95/03067
- WO-A1-95/03067
- US-A- 4 497 801
- US-A- 5 688 771
- CIROUSSEL F ET AL: "PURIFICATION AND PROPERTIES OF AN LD-DIPEPTIDASE FROM BACILLUS SPHAERICUS" INTERNATIONAL JOURNAL OF BIOCHEMISTRY, PERGAMON, vol. 22, no. 5, 1990, pages 525-532, XP008059686 ISSN: 0020-711X
- MEGURO, HIROSHI ET AL: "Study on the Optical Rotatory Dispersion of Di-Peptides and its Application to the Recognition of Di-Peptides from Higher Peptides and Amino Acids" AGR BIOL CHEM, vol. 32, no. 4, 1968, pages 518-521, XP008060334
- AKIO I. ET AL.: 'Lysyl-aspartic acid derivatives as model compounds of growth hormone to induce lipolysis' AGRICULTURAL BIOLOGICAL CHEMISTRY vol. 52, no. 3, 1988, pages 765 - 771, XP001161000
- SETSUNA KINIO ET AL.: 'Studies on biologically active peptide derived from fish and shellfish. 4. Separation and identification of angiotensin 1-concerting enzyme inhibitory peptides from the protease hydrolyzate of undaria pinnatifida protein and their dipeptide derivatives' JOURNAL OF NATIONAL FISHERIES UNIVERSITY vol. 50, no. 2, 2002, pages 61 - 66, XP008059791
- ICHIZO SHINDODA ET AL.: 'A new salty peptide, ornithyl-beta-alanine hydrochloride' PEPTIDE CHEMISTRY, 1983 21ST 1984, pages 43 - 46, XP008059662
- CIROUSSEL FRANCOISE ET AL.: 'Purification and properties of and LD-dipeptidase from bacillus sphaericus' INTERNATIONAL JOURNAL OF BIOCHEMISTRY vol. 22, no. 5, 1990, pages 525 - 532, XP008059686

## Description

### BACKGROUND OF THE INVENTION

### Field of the Invention

The present invention relates to novel positively charged lipids and liposomes comprising the positively charged lipids, more specifically, to positively charged lipids prepared by linking salt forms of acidic amino acid with a variety of lengths of hydrocarbon chain, a process for preparing the lipids, and liposomes comprising the said positively charged lipids.

### Description of the Prior Art

It has been well known that many diseases are generally associated with genetic defects. As an approach to cure the diseases, gene therapy, which is developed for the treatment of disease by allowing the production of normal protein from a gene replacing a disease-causing gene, has been continuously explored in the art. Since the dawn of history, the gene therapy was first attempted to treat ADA(adenosine deaminase) deficiency, and its successful implementation opened an era of gene therapy, which is nowadays being extensively studied worldwide.

On the other hand, the gene delivery system for transferring a desirable foreign gene into a target cell is accompanied with biological and physicochemical means. The biological means using virus, although its gene delivery efficiency is high, has revealed several shortcomings that the introduction of viral genetic material may bring about a side-effect and the size of the delivered gene is rather limited. And, the physicochemical means uses gene gun, injection of naked DNA or liposome. Among them, the method using gene gun or injection of naked DNA is less satisfactory in a sense that the spectrum of target cells are limited, owing to the limitation of transferred region of a foreign gene to the applied spot of gene gun or injection, and the method using liposome has some defects that the gene delivery efficiency is relatively low compared to that employing virus and the expression is temporary. However, liposomes have several advantages as followings: they are capable of forming a lipid-DNA complex easily through the linkage with a gene; the gene delivery efficiency is relatively high compared to the method using gene gun or injection of gene; there is no side-effect caused by the introduction of viral genetic material; there is no limitation in the size of delivered gene; and, the expression of the gene in the target cell can be continuously increased if the liposome is used with a ligand linked with liposome. Accordingly, the application of liposome in the gene therapy is gradually increased, and widely used for the treatment of diseases such as tumor.

For the reasons as above, liposomes such as N-[1-(2,3-dioleyloxy)propyl]-N,N,N-triethylammoniumchloride (DOTMA), N-[1-(2,3-dioleyloxy)propyl]-N,N,N-trimethylammonium methylsulfate(DOTAP), 2,3-dioleyloxy-N-[2-(sperminecarboxyamide) ethyl]-N,N-dimethyl-1-propanammonium trifluoroacetate(DOSPA) and 3β-[N-(N'N'-dimethylaminoethane) carbamoyl] cholesterol(DC-Chol) have been developed so far and have made a great contribution to gene therapy, however, the shortcoming of low efficiency of gene delivery has been regarded as a problem to be overcomed.

Under the circumstances, there are strong reasons for exploring and developing a novel liposome with a higher efficiency of gene delivery.

### SUMMARY OF THE INVENTION

The present inventors have made an effort to develop novel liposomes with a high efficiency of gene delivery, prepared positively charged lipids by employing salt forms of acidic amino acids whose amino groups are protected, and found that liposomes comprising the positively charged lipids can transfer a gene into target cell with a higher efficiency than the liposomes comprising conventional positively charged lipids.

A primary object of the present invention is, therefore, to provide a process for preparing a positively charged lipid by employing a salt form of acidic amino acid.
Another object of the present invention is to provide positively charged lipid prepared by the said process.
The other object of the present invention is to provide a liposome comprising the positively charged lipid.

### BRIEF DESCRIPTION OF THE DRAWINGS

The above and the other objects and features of the present invention will become apparent from the following descriptions given in conjunction with the accompanying drawings, in which:
Figure 1a is a graph showing gene delivery efficiencies of KD-DM/Chol, KD-DM/Dope and KD-DM liposomes with varied amounts of DNA in 293 cell.
Figure 1b is a graph showing gene delivery efficiencies of KD-DP/Chol, KD-DP/Dope and KD-DP liposomes with varied amounts of DNA in 293 cell.
Figure 1c is a graph showing gene delivery efficiencies of KE-DM liposome, and KE-DM/Chol liposomes which were prepared by mixing KE-DM and cholesterol in various ratios, with varied amounts of DNA in 293 cell.
Figure 2a is a graph showing gene delivery efficiencies of KD-DM/Chol, KD-DM/Dope and KD-DM liposomes with varied amounts of DNA in B16BL6 cell.
Figure 2b is a graph showing gene delivery efficiencies of KD-DP/Chol, KD-DP/Dope and KD-DP liposomes with varied amounts of DNA in B16BL6 cell.
Figure 2c is a graph showing gene delivery efficiencies of KE-DM liposome, and KE-DM/Chol liposome which is prepared by mixing KE-DM and cholesterol in a molar ratio of 7:3, with varied amounts of DNA in B16BL6 cell.
Figure 3a is a graph showing gene delivery efficiencies of KD-DM/Chol, KD-DM/Dope and KD-DM liposomes with varied ratios of DNA to liposome in 293 cell.
Figure 3b is a graph showing gene delivery efficiencies of KD-DP/Chol, KD-DP/Dope and KD-DP liposomes with varied ratios of DNA to liposome in 293 cell.
Figure 3c is a graph showing gene delivery efficiencies of KE-DM and KE-DM/Chol liposomes with varied ratios of DNA to liposome in 293 cell.
Figure 4a is a graph showing gene delivery efficiencies of KD-DM/Chol, KD-DM/Dope and KD-DM liposomes with varied ratios of DNA to liposome in B16BL6 cell.
Figure 4b is a graph showing gene delivery efficiencies of KD-DP/Chol, KD-DP/Dope and KD-DP liposomes with varied ratios of DNA to liposome in B16BL6 cell.
Figure 4c is a graph showing gene delivery efficiencies of KE-DM and KE-DM/Chol liposomes with varied ratios of DNA to liposome in B16BL6 cell.

### DETAILED DESCRIPTION OF THE INVENTION

The present invention provides novel positively charged lipids represented by the following general formula (I) : 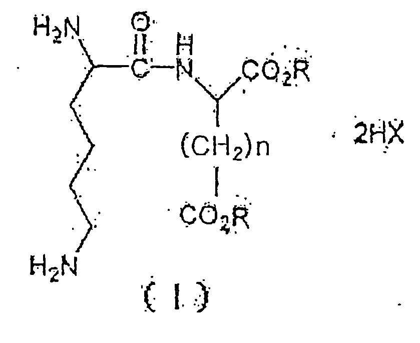 wherein,
R is C₁₀-C₁₈ alkyl or C₁₀-C₁₈ alkenyl;
X is F, Cl, Br, I, CF₃CO₂, HSO₄ or NH₃; and,
n is 1 or 2.

A process for preparing a positively charged lipid comprises the steps of: esterifying or coupling a salt form of an acidic amino acid (II) whose amino group is protected, with an alcoholic compound (III) in the presence of an acidic catalyst of p-toluenesulfonic acid monohydrate or anhydrous toluene to obtain a compound (IV); detaching a protective group from the compound (IV) in the presence of 5 to 20%(w/w) Pd/C or hydrogen in an organic solvent of anhydrous tetrahydrofuran(THF) to obtain a compound (V); coupling the compound (V) with a compound(VI) to obtain a compound (VII) ; reacting the compound (VII) with HCl-dioxane solution, anhydrous dioxane or mixture thereofs at a temperature of 0 to 10°C for 1 to 6hrs, to remove R³ from the compound (VII) to give a compound (VIII) ; and, preparing a salt form of the compound (VIII) to give a positively charged lipid represented as the following general formula (I). 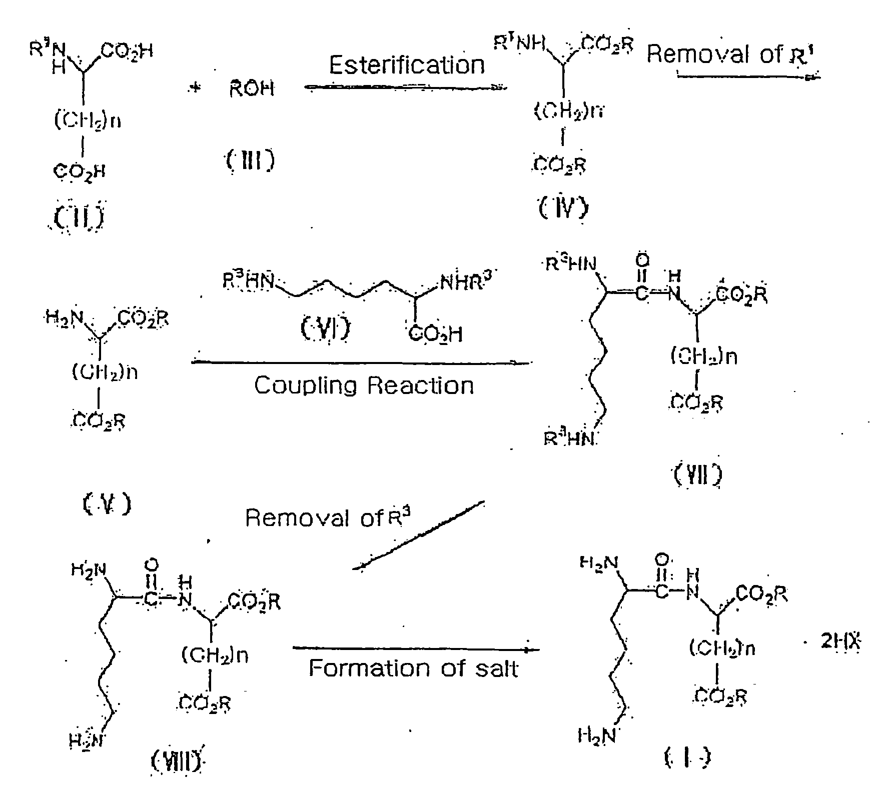 wherein,
R is C₁₀-C₁₈ alkyl or C₁₀-C₁₈ alkenyl;
R¹ and R³ are benzyl carbamate(Cbz), p-methoxybenzyl carbamate (Moz) or p-bromobenzyl carbamate;
X is F, Cl, Br, I, CF₃CO₂, HSO₄ or NH₃; and,
n is 1 or 2.

The process for preparing novel positively charged lipids is further illustrated in more detail, in accordance with the following steps.

### Step 1: Obtainment of compound (IV) from a salt form of acidic amino acid

Compound (IV) is obtained by esterifying or coupling a salt form of an acidic amino acid (II) whose amino group is protected, with an alcoholic compound (III) in the presence of an acidic catalyst of p-toluenesulfonic acid monohydrate or anhydrous toluene: The acidic amino acid includes glutamic acid and aspartic acid, and the coupling reagent includes dicyclohexylcarbodiimide (DCC) and diisopropyldiimide(DIC) . Furthermore, R¹ used to protect the amino group is benzyl carbamate (Cbz), p-methoxybenzyl carbamate (Moz) or p-bromobenzyl carbamate, most preferably benzyl carbamate.

### Step 2: Obtainment of compound (V)

A protective group of the compound (IV) was removed in the presence of 5-20% (w/w) Pd/C or hydrogen in an organic solvent of anhydrous tetrahydrofuran (THF), to obtain a compound (V).

### Step 3: Obtainment of compound (VII) by coupling reaction

The compound(V) is coupled with an activated compound (VI) to obtain compound (VII) : The coupling reaction is performed in an organic solvent of anhydrous dichloromethane at room temperature for 2-4hrs, and amino group of the compound (VII) is protected with R³(=the same as R¹ defined as above).

### Step 4: Obtainment of compound (VIII)

R³ is removed by reacting the compound (VII) with Cl-dioxane solution, anhydrous dioxane or mixture thereofs at the temperature of 0-10°C for 1-6hrs, to obtain a compound (VIII).

### Step 5: Preparation of positively charged lipid

Positively charged lipid represented as general formula(I) is prepared by allowing to give a salt form of the compound (VIII), which is, as described as above, performed concurrently with the removal of R³, preferably by reacting the compound (VIII) with HCl-dioxane solution and anhydrous dioxane at 0-10°C for 1-6hrs, more preferably at 0°C for 2-4hrs, finally to prepare positively charged lipids.

Among the positively charged lipids prepared by the said process, lysine-aspartate-α,Ω-bis(dodecanol)diester, lysine-aspartate-α,Ω-bis(tetradeanol)diester, lysine-aspartate-α,Ω-bis(hexadecanol)diester and lysine-glutamate-α,Ω-bis(tetradecanol)diester, which afford high gene delivery efficiencies, are preferably employed in the invention, and lysine-aspartate-α,Ω-bis(tetradecanol)diester, lysine-aspartate-α,Ω-bis(hexadecanol)diester and lysine-glutamate-α,Ω-bis(tetradecanol)diester are most preferred positively charged lipids, which were named as "KD-DM", "KD-DP" and "KE-DM", respectively.

Liposome comprising positively charged lipid can be prepared by dissolving a mixture of a positively charged lipid and cholesterol or dioleoylphosphatidylethanolamine(DOPE) mixed in a molar ratio of 3:7 to 9:1 in an organic solvent, removing the organic solvent therefrom, hydrating, and mixing at a temperature of 10 to 70°C, where a mixture of chloroform and methanol(2:1, v/v) can be used as the organic solvent. In the present invention, positively charged lipids represented as general formula(I), KD-DM, KD-DP and KE-DM were preferably employed to prepare liposomes such as KD-DM/Chol, KD-DM/DOPE, KD-DP/Chol, KD-DP/DOPE, KE-DM/Chol and KE-DM/DOPE.

DNA-liposome complex was prepared by employing the liposomes thus prepared, and used to deliver a gene into target cell, and examined whether the gene can be expressed efficiently in human kidney cell. The result revealed that: liposomes comprising the positively charged lipids of the present invention provide much higher gene delivery efficiency than the conventional liposome, DOTAP/Chol which is prepared by mixing a previously known lipid of DOTAP (N- [1- (2, 3-dioleoyloxy)propyl]-N,N,N-trimethylammonium methyl sulfate) with cholesterol, and provide similar effect of DNA delivery compared to DOTAP/Chol. Accordingly, positively charged lipids of the present invention can be practically applied for the preparation of liposomes for gene therapy, since they provide better characteristics than the conventional positively charged lipids in terms of gene delivery efficiency and biocompatability.

The present invention is further illustrated in the following examples, which should not be taken to limit the scope of the invention.

### Example 1: Preparation of positively charged lipid KD-DM using aspartic acid

### Example 1-1: Obtainment of compound (IV)

*N*-Cbz-*L*-aspartic acid salt (210mg, 0.8mmol) whose amino group is protected, and tetradecanol were reacted with p-toluenesulfonic acid monohydrate (TsOH · H₂O) (50mg, 0.26mmol) and anhydrous toluene (10mL) for 14hrs at a temperature of 110°C, to obtain a compound (IV) (R=C₁₄H₂₉) with a yield of 62% by the aid of Dean-Stark trap using an eluent (hexane:EtOAc=9.5:0.5, v/v).

¹H NMR (CDCl₃) δ: 0.87-1. 94 (m, 62H), 2.85 (s, 2H) , 4.06(m, 4H), 4.79(m, 1H), 5.12(s, 2H), 5.7(s, 1H), 7.2-7.4 (m, 5H)

### Example 1-2: Obtainment of compound (V)

Protective group Cbz of compound (IV) obtained in Example 1-1 was removed by reacting the compound (300mg, 0.46mmol) with 10% Pd/C(58mg, 0.55mmol) in the presence of hydrogen in an organic solvent of anhydrous tetrahydrofuran(THF) at room temperature, to obtain a compound (V) (R=C₁₄H₂₉) with a yield of 91%.

### Example 1-3: Obtainment of activated compound (VI)

*L*-lysine monohydrochloride (200mg, 1.01mmol) and di-*tert*-butyl dicarbonate (477.9mg, 2.19mmol) were reacted in the presence of NaOH (131.5mg, 3.29memol), THF (3mL) and water(3mL) at room temperature, to give 311mg of compound (VI) (R³=*t*-BOC) with a yield of 82%. Then, the said compound, *N*-hydroxysuccinimide (53mg, 0.46mmol), DCC(103mg, 0.5mmol) and anhydrous dicholomethane were reacted at room temperature, finally to obtain a compound (VI) possessing active group.

### Example 1-4: Obtainment of compound (II)

Compound (V) and compound (VI) obtained in Examples 1-2 and 1-3 were mixed, reacted at room temperature for 3hrs, to obtain a compound (VII) (R=C₁₄H₂₉) with a yield of 61% by aid of Dean-Stark trap using an eluent(hexane:EtOAc = 7:3, v/v).

¹H NMR (CDCl₃) δ : 0.88-2.01 (m, 86H), 2.79-3.05 (m, 4H), 4.10(m, 4H), 4.86 (m, 2H), 6.83 (s, 1H) , 6.9 (s, 1H)

### Example 1-5: Preparation of positively charged lipid

Compound (VII) obtained in Example 1-4 150mg (0.16mmol), HCl 4mL (4.0M dioxane solution) and anhydrous dioxane 4mL were reacted at 0°C, to prepare a positively charged lipid ("KD-DM") with a yield of 94%.

### Example 2: Preparation of positively charged lipid KD-DP using aspartic acid

### Example 2-1: Obtainment of compound (IV)

*N*-Cbz-*L*-aspartic acid salt(210mg, 0.8mmol) whose amino group is protected, hexadecanol (393mg, 1.6mmol), p-toluenesulfonic acid monohydrate (TsOH · H₂O) (50mg, 0.26mmol) and anhydrous toluene 10mL were reacted for 14hrs at 110°C, to obtain a compound (IV) (R=C₁₆H₃₃) with a yield of 65% by aid of Dean-Stark trap using an eluent (hexane:EtOAc=9.5:0.5, v/v).

¹H NMR (CDCl₃) δ : 0.87-1.94 (m, 70H), 2.85 (s, 2H), 4.06(m, 4H), 4.79(m, 1H), 5.12 (s, 2H), 5.7 (s, 1H), 7.2-7.4 (m, 5H)

### Example 2-2: Obtainment of compound ( V )

Protective group Cbz of compound (IV) obtained in Example 2-1 was removed by reacting the compound (300mg, 0.46mmol) with 10% Pd/C(58mg, 0.55mmol) in the presence of hydrogen in an organic solvent of anhydrous tetrahydrofuran(THF) at room temperature, to obtain a compound (V) (R=C₁₆H₃₃) with a yield of 91%.

### Example 2-3: Obtainment of compound (VII)

Compound (V) and compound (VI) obtained in Examples 2-2 and 1-3 were mixed, reacted at room temperature for 3hrs, to obtain a compound (VII) (R=C₁₆H₃₃) with a yield of 62% by the aid of Dean-Stark trap using an eluent (hexane : EtOAc= 7 : 3, v/v).

¹H NMR (CDCl₃) δ : 0.88-2.01 (m, 94H), 2.79-3.05 (m, 4H) , 4.10(m, 4H), 4.86 (m, 2H), 6.83 (s, 1H), 6.9 (s, 1H)

### Example 2-4: Preparation of positively charged lipid

Compound (VII) obtained in Example 2-3 150mg (0.16mmol), HCl 4mL (4.0M dioxane solution) and anhydrous dioxane 4mL were reacted at 0°C, to prepare a positively charged lipid("KD-DP") with a yield of 92%.

### Example 3: Preparation of positively charged lipid KE-DM using glutamic acid

### Example 3-1: Obtainment of compound(IV)

N-Cbz-L-glutamic acid salt(225mg, 0.8mmol) whose amino group is protected, and tetradecanol (343mg, 1.6mmol) were reacted with p-toluenesulfonic acid monohydrate (TsOH · H₂O) (50mg, 0.26mmol) and anhydrous toluene (10mL) for 14hrs at 110°C, to obtain a compound (IV) (R=C₁₄H₂₉) with a yield of 63% by the aid of Dean-stark trap using an eluent (hexane:EtOAc=9.5:0.5, v/v).

¹H NMR (CDCl₃) δ : 0.81-1.9(m, 62H), 2.7-2.85 (m, 4H), 4.04(m, 4H), 4.79(m, 1H), 5.12(s, 2H), 7.2-7.4(m, 5H)

### Example 3-2: Obtainment of compound (V)

Protective group Cbz of compound (IV) obtained in Example 3-1 was removed by reacting the compound (300mg, 0.45mmol) with 10% Pd/C(58mg, 0.55mmol) in the presence of hydrogen in an organic solvent of anhydrous tetrahydrofuran(THF) at room temperature, to obtain a compound(V) (R=C₁₄H₂₉) with a yield of 93%.

### Example 3-3: Obtainment of compound (VII)

Compound(V) and compound (VI) obtained in Examples 3-2 and 1-3 were mixed, reacted at room temperature for 3hrs, to obtain a compound (VII) (R=C₁₄H₂₉) with a yield of 62% by the aid of Dean-Stark trap using eluent (hexane : EtOAc=7 : 3, v/v).

1H NMR (CDCl3) δ : 0.81-2.0 (m, 86H), 2.70-3.05 (m, 6H), 4.04 (m, 4H), 4.8(m, 2H), 6.79(s, 1H), 6.87 (s, 1H)

### Example 3-4: Preparation of positively charged lipid

Compound (VII) obtained in Example 3-3 150mg (0.17mmol), HCl 4mL (4.0M dioxane solution) and anhydrous dioxane 4mL were reacted at 0°C, to prepare a positively charged lipid ("KD-DM") with a yield of 92%.

### Example 4: Preparation of liposomes comprising positively charged lipids

Liposomes of DOTAP/Chol, KD-DM, KD-DM/Chol, KD-DM/DOPE, KD-DP, KD-DP/Chol, KD-DP/DOPE, KE-DM and KE-DM/Chol were prepared by employing positively charged lipids prepared in Examples 1, 2 and 3.

### Example 4-1: Preparation of DOTAP/Chol liposome

DOPAT (Avanti Polar Lipids, USA) and cholesterol(Sigma Chemical Co., USA) were dissolved in a molal ratio of 1:1 in a mixed solvent of chloroform and methanol(2:1, v/v), to prepare DOTAP/Chol liposome. Then, the organic solvent was removed from the lipid containing solution by purging nitrogen gas, trace of organic solvent was subsequently removed by using a vacuum pump, hydrated with deionized distilled water, finally to prepare DOTAP/Chol liposome by the aid of a vortex mixer.

### Example 4-2: Preparation of KD-DM liposome

KD-DM liposome was prepared by the same process as in Example 4-1, with an exception of dissolving KD-DM in a mixed solvent.

### Example 4-3: Preparation of KD-DM/Chol liposome

KD-DM/Chol liposome was prepared by the same process as in Example 4-1, with an exception of dissolving KD-DM and cholesterol in a molal ratio of 1:1 in a mixed solvent.

### Example 4-4: Preparation of KD-DM/DOPE liposome

KD-DM/ DOPE liposome was prepared by the same process as in Example 4-1, with an exception of dissolving KD-DM and dioleoylphosphatidylethanolamine(DOPE, Doosan S.R.L., Korea) in a molal ratio of 1:1 in a mixed solvent.

### Example 4-5: Preparation of KD-DP liposome

KD-DP liposome was prepared by the same process as in Example 4-1, with an exception that KD-DP was dissolved in a mixed solvent and a vortex mixer was used at 65°C.

### Example 4-6: Preparation of KD-DP/Chol liposome

KD-DP/Chol liposome was prepared by the same process as in Example 4-1, with an exception that KD-DP and cholesterol were dissolved in a molal ratio of 1:1 in a mixed solvent and a vortex mixer was used at 65°C.

### Example 4-7: Preparation of KD-DP/DOPE liposome

KD-DP/DOPE liposome was prepared by the same process as in Example 4-1, with an exception that KD-DP and DOPE were dissolved in a molal ratio of 1:1 in a mixed solvent and a vortex mixer was used at 65°C.

### Example 4-8: Preparation of KE-DM liposome

KE-DM liposome was prepared by the same process as in Example 4-1, with an exception of dissolving KE-DM in a mixed solvent.

### Example 4-9: Preparation of KE-DM/Chol liposome

KE-DM/Chol liposome was prepared by the same process as in Example 4-1, with an exception that KE-DM and cholesterol were dissolved in a molal ratio of 3:7, 4:0, 5:5, 6:4, 7:3, 8:2 or 9:1 in a mixed solvent, respectively.

### Example 5: Evaluation of gene delivery efficiency of liposome

Liposomes which were prepared to comprise the varied amounts of DNA(luciferase gene) and varied mixing ratios of DNA:liposome were administered to 293 human kidney cell line(ATCC CRL-1573) and mouse melanoma cell B16BL6, and gene delivery efficiencies were evaluated and compared with one another.

### Example 5-1: Culture of 293 human kidney cell line

293 human kidney cells were inoculated in DMEM(Dulbecco's modified eagle medium, Gibco-BRL, USA) containing 10% (v/v) fetal bovine serum, streptomycin and penicillin, and incubated in an incubator(NAPCO, USA) under an environment of 5% (v/v) CO₂ at 37°C, which were then aliquoted in 24-well plate to reach the cell density of 1x10⁵ cell/well or 5x10⁴ cell/well, and further incubated for 24hrs to stabilize the cells.

### Example 5-2: Culture of mouse melanoma cell B16BL6

Mouse melanoma cells B16BL6293 were inoculated in MEM(minimum essential medium, Gibco-BRL, USA) containing 5%(v/v) fetal bovine serum, streptomycin and penicillin, and incubated in an incubator(NAPCO, USA) under an environment of 5% (v/v) CO₂ at 37°C, which were aliquoted in 24-well plate to reach the cell density of 1x10⁵ cell/well or 5x10⁴ cell/well, and further incubated for 24hrs to stabilize the cells.

### Example 5-3: Evaluation of gene delivery efficiency with varied amounts of DNA

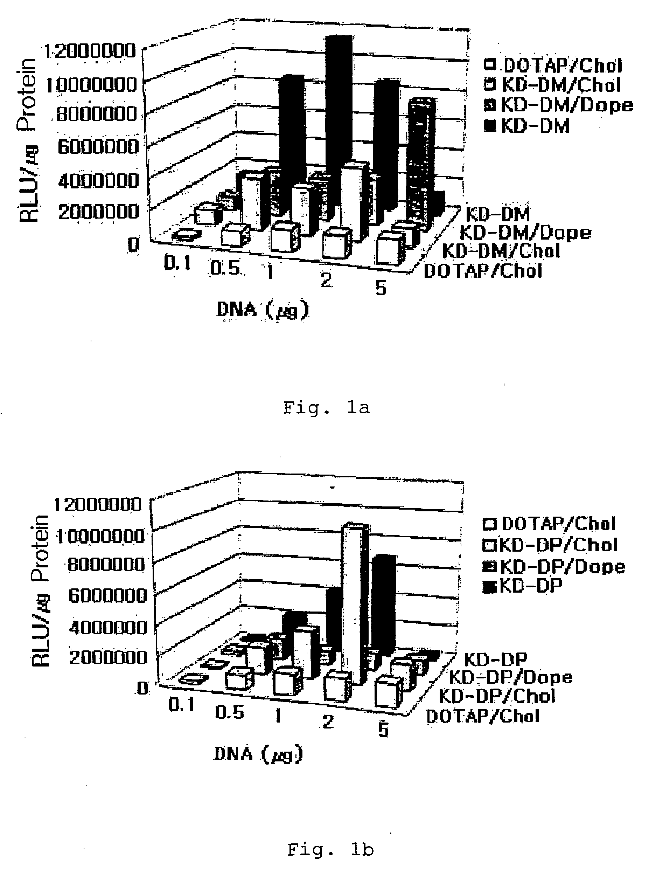

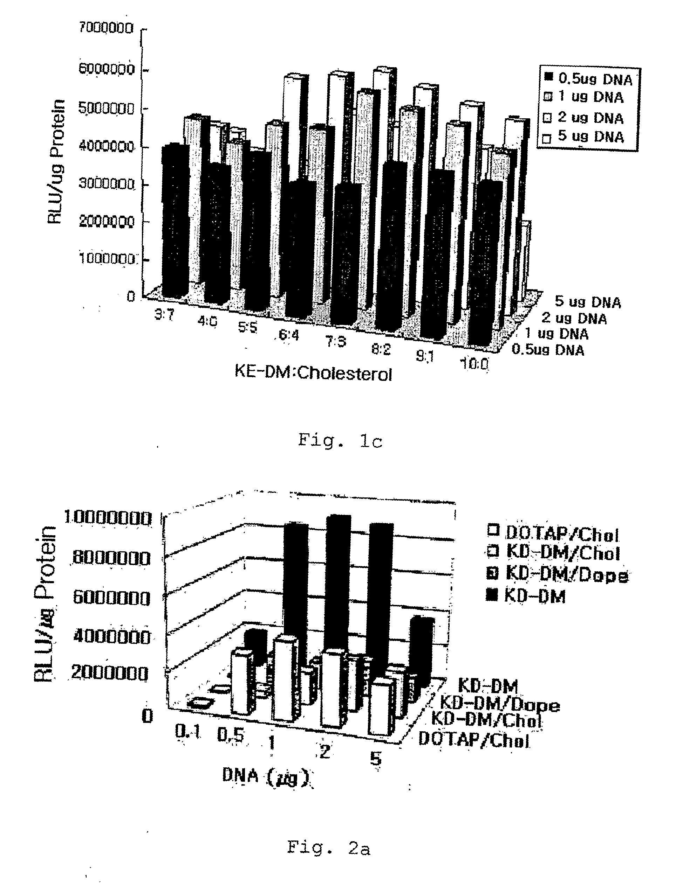

For the evaluation of gene delivery efficiency of liposomes with varied amounts of DNA, DNA-liposome complex was prepared by mixing DNA and liposome(1:12, w/w) in 500 µℓ of serum-free medium, while varying the amounts of luciferase gene as 0.5 µg, 1 µg, 2 µg and 5 µg. 500 µℓ of the DNA-liposome complex was aliquoted to each well containing 293 kidney cells or B16BL6 cells, and incubated in an incubator under an environment of 5% (v/v) CO₂ at 37°C for 4hrs, then changed the medium to a fresh medium containing serum and incubated for 48hrs. After incubation, media were discarded, cells were lysed by adding 100 µℓ of Luciferase cell culture lysis reagent(Promega, USA), and centrifuged at 14000rpm for 10sec. And then, 10 µℓ of supernatant was mixed with 20 µℓ of a substrate of luciferase, and luminescence was measured by using Luminometer(Mini lumat LB9506, EG&G BERTHOLD). Proteins in each well were quantitated by DC protein assay(Bio-rad, USA) (see: Figures 1a-1c and 2a-2c).

Figures 1a and ib are graphs showing gene delivery efficiencies of KD-DM/Dope and KD-DM liposomes, and KD-DP/Dope and KD-DP liposomes with varied amounts of DNA in 293 cells, respectively, and Figure 1c is a graph showing gene delivery efficiencies of KE-DM liposome, and KE-DM/Chol liposomes prepared by mixing KE-DM and cholesterol in various ratios, with varied amounts of DNA in 293 cells.

As shown in Figures 1a and 1b, luciferase gene delivery efficiencies of liposomes comprising positively charged lipids were higher than that of conventional DOTAP/Chol liposome. Comparison with the conventional DOTAP/Chol revealed that the amounts of DNA showing the highest gene delivery efficiency of each liposome were: 1 µg for KD-DM, 2 µg for KD-DM/Chol, 5 µg for KD-DM/DOPE, 2 µg for KD-DP, 2 µg for KD-DP/Chol, 2 µg for KE-DM, and 2 µg for KE-DM/Chol. Especially, as shown in Figure 1c, the mixing ratio of KE-DM to cholesterol showing the highest gene delivery efficiency of each liposome was 7:3(in molar ratio).

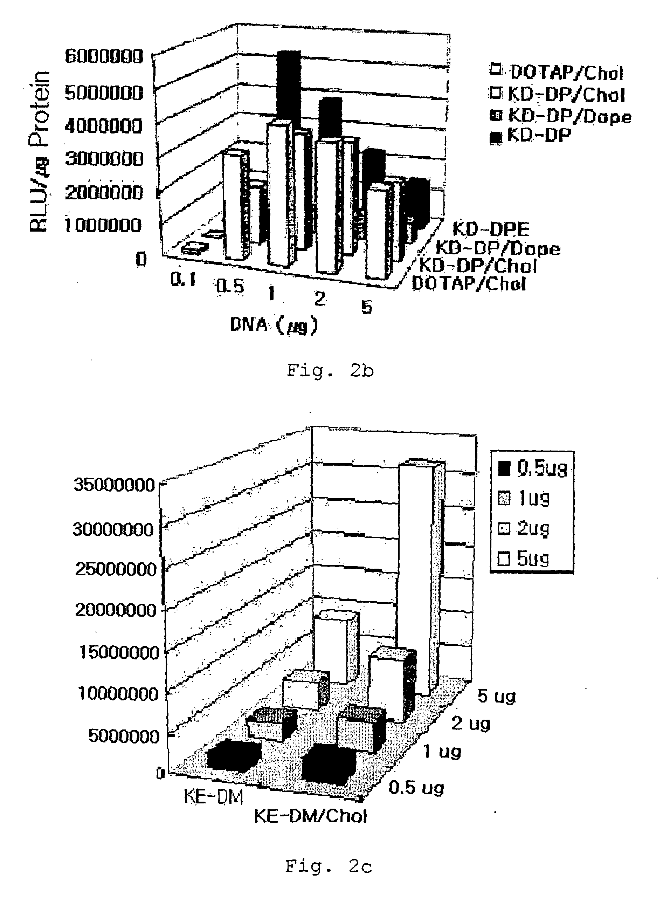

Figures 2a and 2b are graphs showing gene delivery efficiencies of KD-DM/Chol, KD-DM/Dope and KD-DM liposomes, and KD-DP/Chol, KD-DP/Dope and KD-DP liposomes with varied amounts of DNA in B16BL6 cells, respectively, and Figure 2c is a graph showing gene delivery efficiencies of KE-DM liposome and KE-DM/Chol liposome which is prepared by mixing KE-DM and cholesterol in a molar ratio of 7:3, with varied amounts of DNA in B16BL6 cell.

As shown in Figures 2a and 2b, among the positively charged lipids of the present invention, positively charged liposomes using aspartic acid showed gene delivery efficiencies lower than the conventional DOTAP/Chol liposome in case of cholesterol-bound liposome, and higher than the conventional DOTAP/Chol in case of cholesterol-unbound liposome. To the contrary, as shown in Figure 2c, in case of positively charged liposomes using glutamic acid, cholesterol-bound liposome showed gene delivery efficiencies higher than cholesterol-unbound liposome. And, the amounts of DNA showing the highest gene delivery efficiency of each liposome were: 1 µg for KD-DM, 0.5 µg for KD-DP and 5 µg for KE-DM/Chol.

### Example 5-4: Measurement of gene delivery efficiency with varied mixing ratio of DNA:liposome

For the evaluation of gene delivery efficiency with varied mixing ratio of DNA:liposome, DNA-liposome complexes were prepared by the same method as in Example 5-3 except that 1 µg of luciferase gene was used and the mixing ratios of DNA:liposome were 1:3, 1:6, 1:9, 1:12 and 1:18(w/w), and then, added to 293 kidney cells and incubated for 48hrs. After incubation, the media were discarded, the cells were lysed by adding 100 µℓ of Luciferase cell culture lysis reagent (Promega, USA), then centrifuged at 14000rpm for 10sec. Subsequently, 10 µℓ of supernatant was mixed with 20 µℓ of a substrate of luciferase, and luminescence was measured by using Luminometer(Mini lumat LB9506, EG&G BERTHOLD) and proteins in each well were quantitated by DC protein assay(Bio-rad, USA).

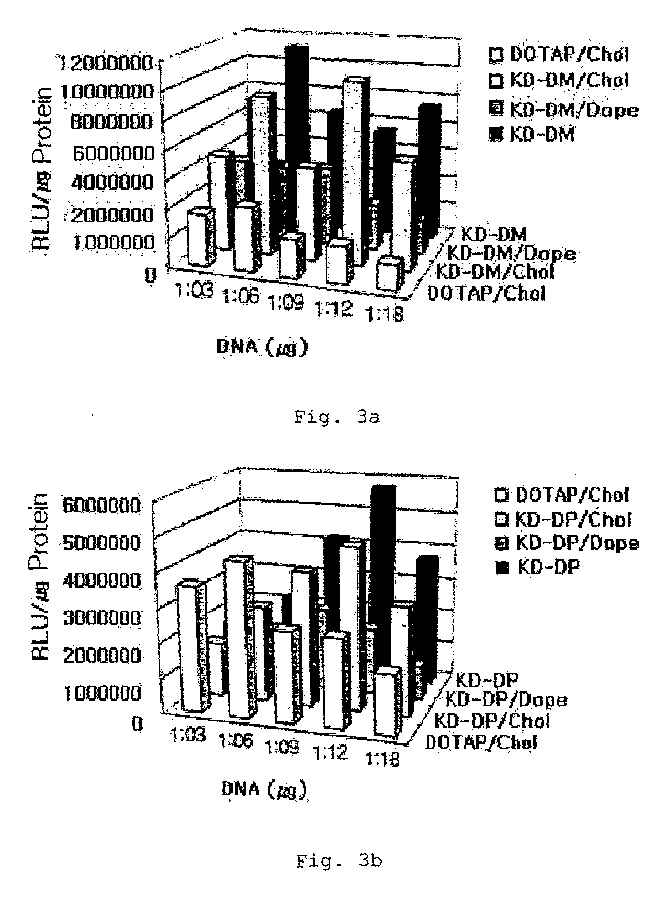

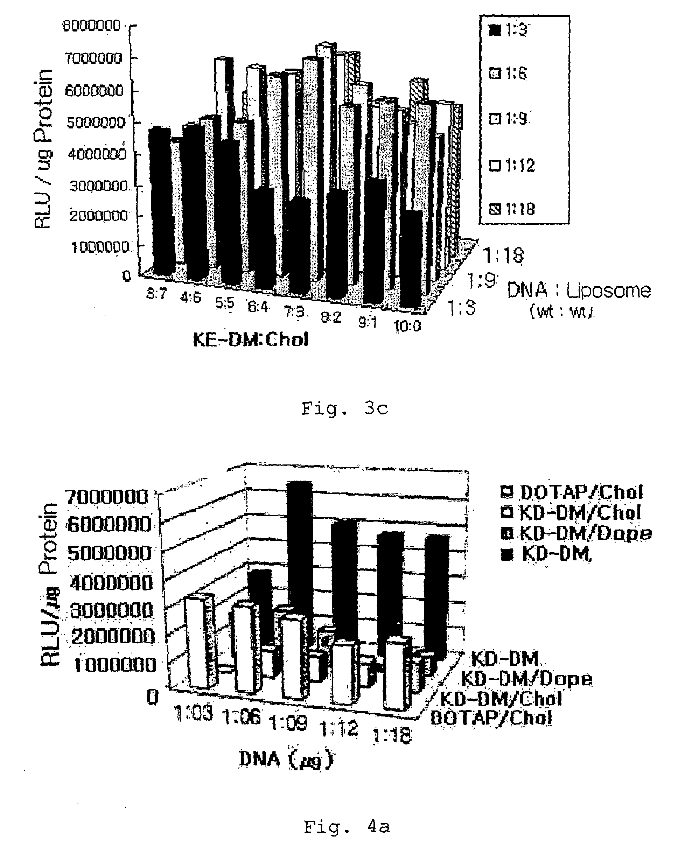

Figures 3a, 3b and 3c are graphs showing gene delivery efficiencies of KD-DM/Chol, KD-DM/Dope and KD-DM liposomes, KD-DP/Chol, KD-DP/DOPE and KD-DP liposomes, and KE-DM and KE-DM/Chol liposomes with varied ratios of DNA to liposome in 293 cells, respectively.

As shown in Figures 3a and 3b, the ratios of DNA to liposome prepared by employing aspartic acid showing the highest gene delivery efficiency were: 1:6(w/w) for KD-DM, 1:12(w/w) for KD-DM/Chol, 1:6(w/w) for KD-DM/DOPE, 1:12(w/w) for KD-DP, and 1:12(w/w) for KD-DP/Chol. As shown in Figure 3c, in the case of liposome prepared by employing glutamic acid, the mixing ratio showing the highest efficiency was 7:3(in molar ratio) of KE-DM:cholesterol and 1:9(w/w) of DNA:liposome.

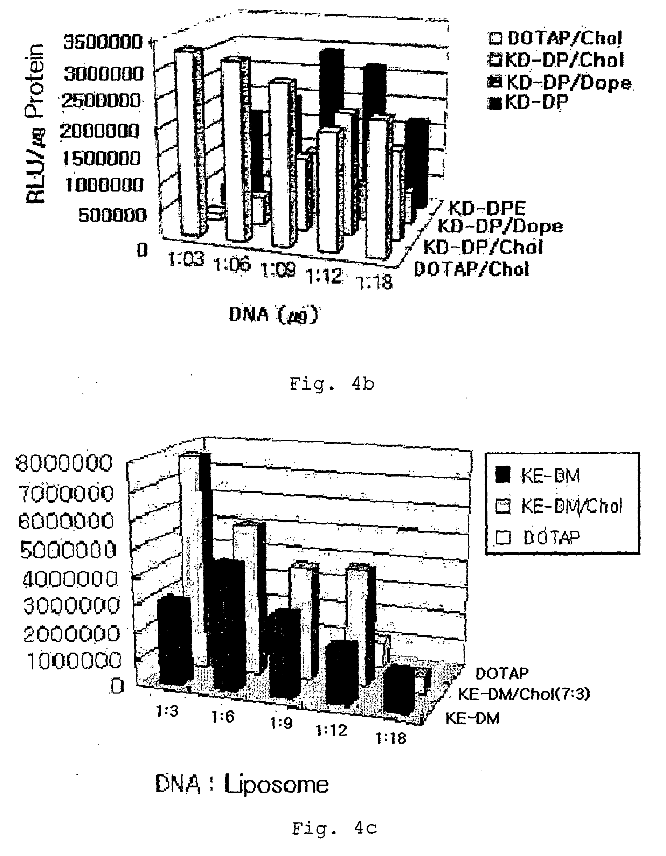

Figures 4a, 4b and 4c are graphs showing gene delivery efficiencies of KD-DM/Chol, KD-DM/Dope and KD-DM liposomes, KD-DP/Chol, KD-DP/DOPE and KD-DP liposomes, and KE-DM and KE-DM/Chol liposomes with varied ratios of DNA to liposome in B16BL6 cells, respectively.

As shown in Figures 4a and 4b, the ratios of DNA to liposome prepared by employing aspartic acid showing the highest gene delivery efficiency were: 1:6(w/w) for KD-DM and 1:9(w/w) for KD-DP. As shown in Figure 4c, in the case of liposome prepared by employing glutamic acid, the ratio showing the highest efficiency was 7:3(in molar ratio) of KE-DM:cholesterol and 1:3 (w/w) of DNA:liposome.

As clearly illustrated and demonstrated as above, the present invention provides positively charged lipids prepared by linking salt forms of acidic amino acid with a variety of lengths of hydrocarbon chain, a process for preparing the lipids, and liposomes comprising the said positively charged lipids. Positively charged lipids of the invention can be practically applied for the preparation of liposomes for gene therapy, since they provide better characteristics than the conventional positively charged lipids in terms of gene delivery efficiency and biocompatibility.

## Claims

1. A positively charged lipid of formula (I): 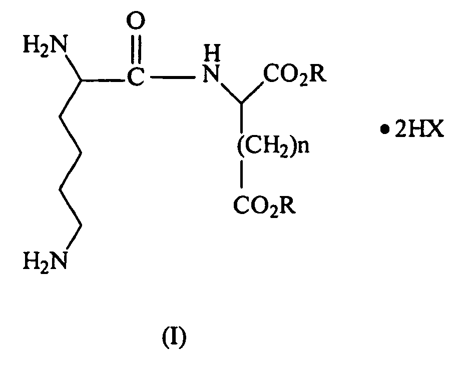 wherein,
R is C₁₀-C₁₈ alkyl or C₁₀-C₁₈ alkenyl;
X is F, Cl, Br, I, CF₃CO₂, HSO₄ or NH₃; and,
n is 1 or 2.

2. A positively charged lipid according to claim 1, wherein the positively charged lipid is lysine-aspartate-α,Ω-bis(tetradecanol)diester (KD-DM), lysine-aspartate-α,Ω-bis(hexadecanol)diester (KD-DP) or lysine-glutamate-α,Ω-bis(tetradecanol)diester (KE-DM).

3. A process for preparing a positively charged lipid according to claim 1 or 2 which comprises the steps of:
(i) esterifying or coupling a salt of an acidic amino acid (II) having a protected amino group protected by a group R¹, with an alcoholic compound (III) in the presence of an acidic catalyst of p-toluenesulfonic acid monohydrate or anhydrous toluene to obtain a compound (IV);
(ii) detaching protective group R¹ from the compound (IV) in the presence of 5 to 20% (w/w) Pd/C or hydrogen in an organic solvent of anhydrous tetrahydrofuran (THF) to obtain a compound (V);
(iii) coupling the compound (V) with a compound (VI) to obtain a compound (VII);
(iv) removing the group R³ from the compound (VII) in HCl-dioxane solution, anhydrous dioxane or mixtures thereof at a temperature of 0 to 10°C for 1 to 6hrs, to obtain a compound (VIII); and,
(v) converting the compound (VIII) to a salt to give a positively charged lipid of the formula (I) 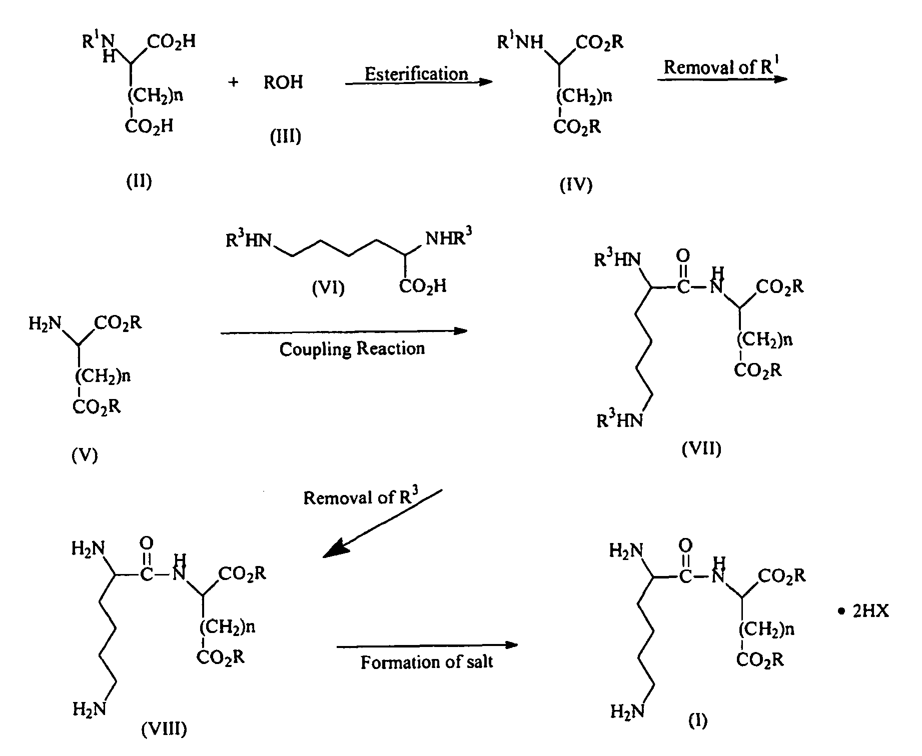 wherein
R is C₁₀-C₁₈ alkyl or C₁₀-C₁₈ alkenyl;
R¹ and R³ are benzyl carbamate (Cbz), p-methoxybenzyl carbamate (Moz) or p-bromobenzyl carbamate;
X is F, C1, Br, I, CF₃CO₂, HSO₄ or NH₃; and,
n is 1 or 2.

4. A process according to claim 3, wherein the acidic amino acid of formula (II) is glutamic acid or aspartic acid.

5. A process according to claim 3 or 4, wherein the coupling reaction in step (iii) is performed at room temperature for 2 to 4 hours in an organic solvent of anhydrous dichloromethane.

6. A process for preparing a liposome comprising a positively charged lipid, which comprises the steps of:
dissolving a mixture of a positively charged lipid of claim 1 or 2 and cholesterol or dioleoylphosphatidylethanolamine (DOPE) mixed in a molar ratio of 3:7 to 9:1 in an organic solvent; and,
removing the organic solvent from the mixture, hydrating and mixing at a temperature of 10 to 70°C.

7. A process according to claim 6, wherein the organic solvent is a mixture of chloroform and methanol (2:1, v/v).

8. A liposome comprising a positively charged lipid according to claim 1 or 2.

9. A liposome according to claim 8 which is obtainable by a process according to claim 6 or 7.

10. A liposome according to claim 9 which comprises lysine-glutamate-α,Ω-bis(tetradecanol)diester (KE-DM) and cholesterol in a molar ratio of 7:3.

## Patentansprüche

1. Positiv geladenes Lipid der Formel (I): 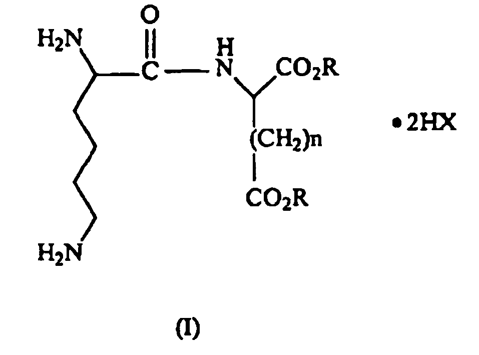 wobei
R C₁₀-C₁₈-Alkyl oder C₁₀-C₁₈-Alkenyl bedeutet;
X F, Cl, Br, I, CF₃CO₂, HSO₄ oder NH₃ bedeutet; und
n den Wert 1 oder 2 hat.

2. Positiv geladenes Lipid nach Anspruch 1, wobei es sich beim positiv geladenen Lipid um Lysin-aspartat-α,Ω-bis-(tetradecanol)-diester (KD-DM), Lysin-aspartat-α,Ω-bis-(hexadecanol)-diester (KD-DP) oder Lysin-glutamat-α,Ω-bis-(tetradecanol)-diester (KE-DM) handelt.

3. Verfahren zur Herstellung eines positiv geladenen Lipids nach Anspruch 1 oder 2, umfassend die folgenden Stufen:
(i) das Verestern oder Kuppeln eines Salzes einer sauren Aminosäure (II), die eine durch eine Gruppe R¹ geschützte Aminogruppe aufweist, mit einer alkoholischen Verbindung (III) in Gegenwart eines sauren Katalysators in Form von p-Toluolsulfonsäure-monohydrat oder von wasserfreiem Toluol unter Bildung einer Verbindung (IV);
(ii) das Ablösen der Schutzgruppe R¹ von der Verbindung (IV) in Gegenwart von 5 bis 20 % (Gew./Gew.) Pd/C oder Wasserstoff in einem organischen Lösungsmittel in Form von wasserfreiem Tetrahydrofuran (THF) unter Bildung einer Verbindung (V);
(iii) das Kuppeln der Verbindung (V) mit einer Verbindung (VI) unter Bildung einer Verbindung (VII);
(iv) das Entfernen der Gruppe R³ von der Verbindung (VII) in HCl-Dioxan-Lösung, wasserfreiem Dioxan oder Gemischen davon bei einer Temperatur von 0 bis 10 °C für 1 bis 6 Stunden unter Bildung einer Verbindung (VIII); und
(v) das Umwandeln der Verbindung (VIII) in ein Salz unter Bildung eines positiv geladenen Lipids der Formel (I) 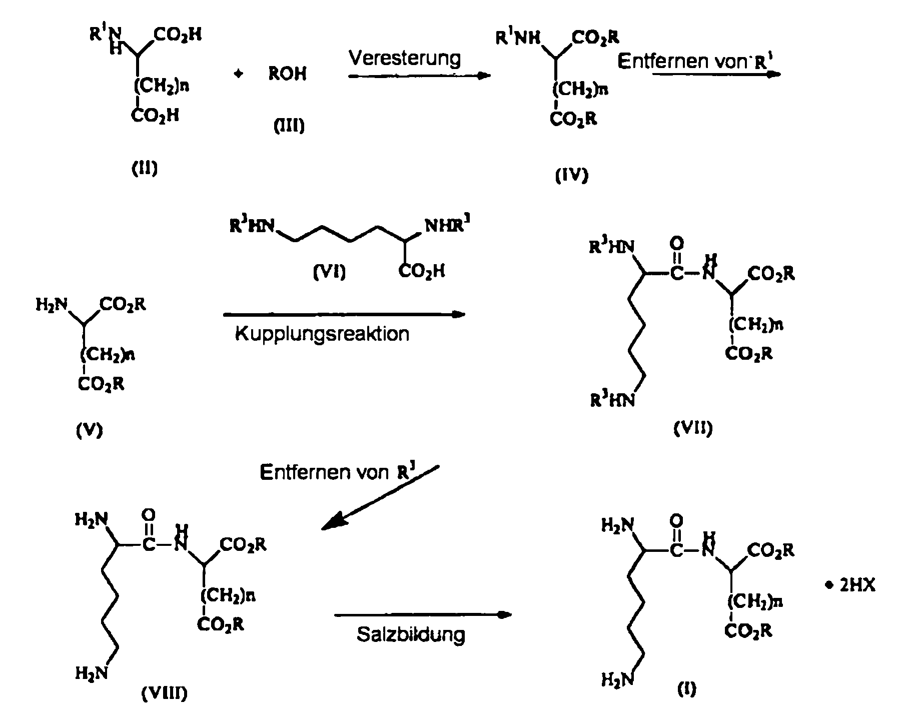 wobei
R C₁₀-C₁₈-Alkyl oder C₁₀-C₁₈-Alkenyl bedeutet;
R¹ und R³ Benzylcarbamat (Cbz), p-Methoxybenzylcarbamat (Moz) oder p-Brombenzylcarbamat bedeuten;
X F, Cl, Br, I, CF₃CO₂, HSO₄ oder NH₃ bedeutet; und
n den Wert 1 oder 2 hat.

4. Verfahren nach Anspruch 3, wobei es sich bei der sauren Aminosäure der Formel (II) um Glutaminsäure oder Asparginsäure handelt.

5. Verfahren nach Anspruch 3 oder 4, wobei die Kupplungsreaktion in Stufe (iii) bei Raumtemperatur für 2 bis 4 Stunden in einem organischen Lösungsmittel in Form von wasserfreiem Dichlormethan durchgeführt wird.

6. Verfahren zur Herstellung eines Liposoms, das ein positiv geladenes Lipid umfasst, umfassend die folgenden Stufen:
das Lösen eines Gemisches aus einem positiv geladenen Lipid nach Anspruch 1 oder 2 und Cholesterin oder Dioleoylphosphatidylethanolamin (DOPE), die in einem Molverhältnis von 3:7 bis 9:1 in einem organischen Lösungsmittel vermischt sind; und
das Entfernen des organischen Lösungsmittels aus dem Gemisch, das Hydratisieren und das Vermischen bei einer Temperatur von 10 bis 70 °C.

7. Verfahren nach Anspruch 6, wobei es sich beim organischen Lösungsmittel um ein Gemisch aus Chloroform und Methanol (2:1, Vol./Vol.) handelt.

8. Liposom, umfassend ein positiv geladenes Lipid nach Anspruch 1 oder 2.

9. Liposom nach Anspruch 8, das durch ein Verfahren gemäß Anspruch 6 oder 7 erhältlich ist.

10. Liposom nach Anspruch 9, das Lysin-glutamat-α,Ω-bis-(tetradecanol)-diester (KE-DM) und Cholesterin in einem Molverhältnis von 7:3 umfasst.

## Revendications

1. Lipide à charge positive, de formule (1) : 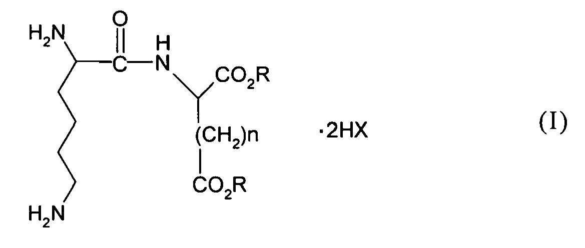 dans laquelle :
- R représente un groupe alkyle en C₁₀₋₁₈ ou alcényle en C₁₀₋₁₈ ;
- X représente un atome de fluor, de chlore, de brome ou d'iode ou un groupe de formule CF₃CO₂, HSO₄ ou NH₃ ;
- et l'indice n vaut 1 ou 2.

2. Lipide à charge positive, conforme à la revendication 1, lequel lipide à charge positive est de l'α,ω-diester lysine-aspartate de bis(tétradécanol) (KD-DM), de l'α,ω-diester lysine-aspartate de bis(hexadécanol) (KD-DP) ou de l'α,ω-diester lysine-glutamate de bis(tétradécanol) (KE-DM).

3. Procédé de préparation d'un lipide à charge positive, conforme à la revendication 1 ou 2, lequel procédé comporte les étapes suivantes :
i) estérifier ou coupler un sel d'un acide aminé à caractère acide de formule (II), dont le groupe amino est protégé par un groupe symbolisé par R¹, avec un alcool de formule (III), en présence d'un catalyseur acide, qui est du monohydrate d'acide para-toluènesulfonique, et de toluène anhydre, de manière à obtenir un composé de formule (IV) ;
ii) éliminer du composé (IV) le groupe protecteur R¹, en présence d'hydrogène et de charbon palladié à 5 à 20 % en poids, dans un solvant organique qui est du tétrahydrofurane (THF) anhydre, de manière à obtenir un composé de formule (V) ;
iii) coupler ce composé (V) avec un composé de formule (VI), de manière à obtenir un composé de formule (VII) ;
iv) éliminer de ce composé (VII) les groupes symbolisés par R³, dans du dioxane anhydre, dans une solution de chlorure d'hydrogène dans du dioxane, ou dans un mélange de tels solvants, et à une température de 0 à 10 °C pendant 1 à 6 heures, de manière à obtenir un composé de formule (VIII) ;
v) et convertir ce composé (VIII) en un sel, de manière à obtenir un lipide à charge positive, de formule (I) : 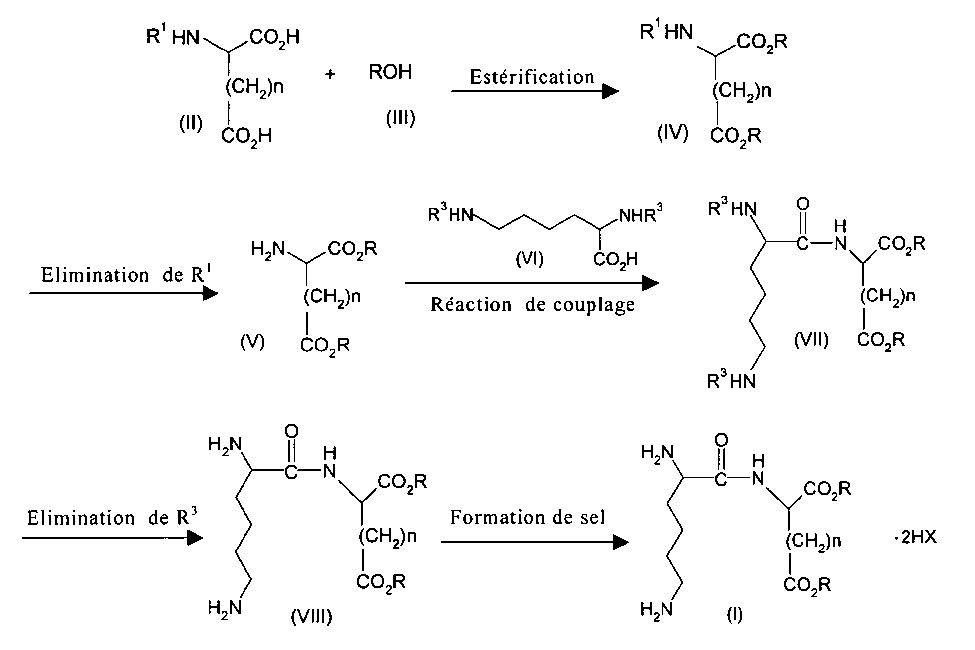 dans lesquelles formules :
- R représente un groupe alkyle en C₁₀₋₁₈ ou alcényle en C₁₀₋₁₈ ;
- R¹ et R³ représentent chacun un groupe benzylcarbonyloxy (Cbz), para-méthoxy-benzylcarbonyloxy (Moz) ou para-bromo-benzylcarbonyloxy, donnant le carbamate correspondant ;
- X représente un atome de fluor, de chlore, de brome ou d'iode ou un groupe de formule CF₃CO₂, HSO₄ ou NH₃ ;
- et l'indice n vaut 1 ou 2.

4. Procédé conforme à la revendication 3, dans lequel l'acide aminé à caractère acide de formule (II) est de l'acide glutamique ou de l'acide aspartique.

5. Procédé conforme à la revendication 3 ou 4, dans lequel la réaction de couplage de l'étape (iii) est effectuée à température ambiante, dans un solvant organique qui est du dichlorométhane anhydre, et dure de 2 à 4 heures.

6. Procédé de préparation d'un liposome comprenant un lipide à charge positive, lequel procédé comporte les étapes suivantes :
- dissoudre un mélange d'un lipide à charge positive, conforme à la revendication 1 ou 2, et de cholestérol ou de dioléoyl-phosphatidyl-éthanolamine (DOPE), mélangés en un rapport molaire de 3/7 à 9/1, dans un solvant organique ;
- et chasser du mélange le solvant organique, hydrater le résidu, puis brasser le tout à une température de 10 à 70 °C.

7. Procédé conforme à la revendication 6, dans lequel le solvant organique est un mélange 2/1 v/v de chloroforme et de méthanol.

8. Liposome comprenant un lipide à charge positive, conforme à la revendication 1 ou 2.

9. Liposome conforme à la revendication 8, qu'on peut obtenir par un procédé conforme à la revendication 6 ou 7.

10. Liposome conforme à la revendication 9, qui comprend de l'α,ω-diester lysine-glutamate de bis(tétradécanol) (KE-DM) et du cholestérol en un rapport molaire de 7/3.
